# EUROPÄISCHE PATENTSCHRIFT

(11) **EP 0 516 190 B1**
(45) Veröffentlichungstag und Bekanntmachung des Hinweises auf die Patenterteilung: **24.07.2002**
(21) Anmeldenummer: 92113308.8
(22) Anmeldetag: 26.04.1985
(51) Int. Cl.: C12N 15/02, C12P 21/08, C07K 16/00, A61K 39/395, G01N 33/577

(54) **Monoklonale Antikörper, Verfahren zu ihrer Herstellung sowie ihre Verwendung**
Monoclonal antibodies, process for preparing them and their use
Anticorps monoclonaux, leur procédé de préparation et leur application

(30) Priorität: 07.05.1984 DE 3416774
(43) Veröffentlichungstag der Anmeldung: 02.12.1992
(62) Teilanmeldung aus: 85105083.1
(73) Patentinhaber: Bosslet, Klaus, Dr., 13465 Berlin (DE)
(72) Erfinder: Bosslet,Klaus, W-3550 Marburg (DE); Sedlacek,Hans-Harald, W-3550 Marburg (DE)
(74) Vertreter: Weiss, Wolfgang, Dipl.-Chem. Dr.

(56) Entgegenhaltungen:
- EP-A- 0 098 162
- EP-A- 0 141 079
- WO-A-81/01469
- WO-A-84/02983
- PROCEEDINGS OF THE NATL. ACADEMY OF SCIENCES USA, Band 77, Nr. 1, Januar 1980 Washington, DC (US); R.S. ACCOLLA et al., Seiten 563-566
- CHEMICAL ABSTRACTS, Band 94, Nr. 17, 27 April 1981, Columbus, OH (US); K.F. MITCHELL, Seite 577, Nr. 137538s

## Beschreibung

Die Erfindung betrifft einen monoklonalen Antikörper (MAK), der an definierte zytoplasmatische, membranasoziierte und sezerniete Antigene bindet gekennzeichnet durch die Merkmale gemäß Anspruch 1. Der Antikörper kann wie andere ähnliche Antikörper als Diagnostikum oder als Wirkstoff bzw. als Wirkstoffträger verwendet werden.

Die Technik der Immunisierung mit definierten, isolierten Antigenen und der Produktion von Antikörpern gegen solche Antigene ist bekannt. Es ist auch bekannt, mit ungereinigtem Antigenmaterial zu immunisieren und diejenigen Antikörper zu selektieren, die eine bestimmte Komponente einer solchen Antigenmischung erkennen.

Auf Grund dieser Gegebenheiten hat der Erfinder beim an sich bekannten Induzieren von verschiedenen Antikörpern gefunden, dass ein von ihm neuselektierter monoklonaler Antikörper wenigstens mit Glykoproteinen vom MW 180 ± 10 KD und MW 95 ± 10 KD bzw. bestimmten Epitopen auf diesen Proteinantigenen reagiert.

Ein CEA-verwandtes Protein ist beispielsweise in einer Colonkarzinomzelllinie (DE-TA) mittels Immunpräzipitation nachweisbar; es besitzt ein signifikant geringeres Molekulargewicht als die in der Literatur beschriebenen Molekulargewichte für CEA. Das 180 KD Protein ist aufgrund starker Kreuzreaktion mit heterologen anticarcinoembryonalen Antigen-Antiseren dem 1965 von Gold & Freedman (J. exp. Med. (1965), 122, 467) isolierten carcinoembryonalen Antigen (CEA) sehr ähnlich. Auf diesem komplexen Glykoprotein befinden sich Epitope, die auch auf anderen nicht mit CEA identischen Molekülen vorkommen. Es handelt sich um die Antigene NCA₂, (NCA-95 - nonspecific crossreacting antigen; J. Immun. III (1973), 1926), NCA₁ (nonspecific cross reacting Antigen; Ann. N.Y. Acad. Sci. (1975), 259, 389), BGP-I (Int. J. Cancer (1976, 17, 588) und CEA_{low} (Scand. J. Immunol. (1978), Vol. 8, Suppl. 8, 423-428).

Der erfindungsgemässe monoklonale Antikörper zeigt eine spezifische Kreuzreaktion mit dem granulozyten-assozierten Protein NCA₂ (NCA-95). Die ausgezeichneten Eigenschaften und Möglichkeiten dieses gegenüber dem Stand der Wissenschaft neuen monoklonalen Antikörpers wurden bisher nicht erkannt und gewürdigt.

Weiterer wesentlicher Gegenstand der Erfindung ist eine vorteilhafte Verfahrensweise zur Herstellung des erfindungsgemässen monoklonalen Antikörpers. Hierzu werden Säugetiere, in erster Linie Mäuse mit Zellen eines CEA-positiven Adenokarzinoms der Lunge oder mit kommerziell erhältlichem carcinoembryonalen Antigen (CEA) immunisiert, Milzzellen aus einem solchen immunisierten Tier entnommen, mit der Zelllinie X 63-Ag8653 fusioniert, die Hybridome - wie im folgenden beschrieben - selektioniert und daraus der erfindungsgemässe monoklonale Antikörper gewonnen.

Die nach der Fusion von Milzzellen aus immunisierten Tieren und der Zelllinie X 63 - Ag 8653 entstandenen Hybridome werden getestet, ob sie den Antikörper dieser gewünschten Spezifität enthalten. Unter den ausgetesteten Hybridomüberständen haben sich erfahrungsgemäss stets eine Anzahl gefunden, welche Antikörper der oben beschriebenen Spezifitäten enthalten. Diese Antikörper sezernierenden Hybridome werden kloniert und die von diesen Hybridomaklonen gewonnenen monoklonalen Antiköper benutzt, um das von ihnen erkannte Antigen immunchemisch zu charakterisieren. Das Molekulargewicht wird bestimmt im Vergleich zu kommerziell erhältlichen Markern. Die Antigene können dann affinitäts-chromatographisch gereinigt werden.

Zusätzlich wird die Bindung der monoklonalen Antikörper an histologische Präparate mittels der indirekten Immunperoxidasetechnik gemessen (J. Clin. Pathol. (1979), 32, 971).

Weiterhin wird die molekulare Charakterisierung der von dem monoklonalen Antikörper erkannten Antigene bzw. Antigenmolekülsubpopulationen zusätzlich zur Westernblotanalyse mittels Radioimmunpräzipitation durchgeführt. (Behring Institute Mitteilungen (1984), 74, 27-34).

Die molekulare Charakterisierung der von den MAK erkannten Epitope auf humanem Gewebe erfolgte wie im folgenden beschrieben:
4 - 6 µm dicke Schnitte kryopräservierter humaner, die entsprechenden Epitope tragenden Gewebe wurden nach dem Antrocknen auf dem Objektträger (30 - 60 min bei Raumtemperatur) für 1 - 2 sec in Aceton fixiert. Anschliessend werden sie
a) für die Perjodatoxidation in PBS pH 7,2 mit 1 g/l BSA 10 min gewaschen, kurz in PBS pH 7.2 eingetaucht (gespült) und 1 Stunde bei Raumtemperatur in der feuchten Kammer mit 1g/100 ml Perjodsäure in PBS pH 7,2 inkubiert. Anschliessend wird 3 x 5 min in 1 g/l BSA in PBS pH 7,2 gewaschen und die Schnitte mit Ziege-Normalserum inkubiert, wie in J. Clin. Pathol. (1979) 32, 971 mit den zu testenden MAK weiterbehandelt und deren Bindung an die Gewebe mittels der indirekten Immunperoxidasetechnik nachgewiesen.
b) für die Neuraminidasebehandlung 2 x 10 min in 0,05 M Na⁺, CA⁺⁺ Acetatpuffer pH 5,5 gewaschen und 1 Std. mit verschiedenen Mengen von Vibrio cholerae Neuraminidase (100 U/ml, 10 U/ml, 1 U/ml, 0,1 U/ml in obigem Acetatpuffer bei Raumtemperatur in der feuchten Kammer inkubiert. Nach zweimaligem Waschen in Acetatpuffer und zweimaligem Waschen in PBS pH 7,2 mit 1 g/l BSA werden die Schnitte mit Ziege-Normalserum inkubiert, wie in J. Clin. Pathol. (1979) 32, 971 mit den zu testenden MAK weiterbehandelt und deren Bindung an die Gewebe mittels der indirekten Immunperoxidasetechnik nachgewiesen.

Anstelle von 4 - 6 µm dicken kryopräservierten Gewebeschnitten können zur Austestung der Formaldehyd- und Paraffinresistenz des von dem erfindungsgemässen Antikörper erkannten Epitops, in Formaldehyd fixierte und paraffineingebettete Gewebe genommen werden, die in einem Mikrotom geschnitten, deparaffiniert (B. Romeins, Mikroskopische Techniken, 16 Auflage, S. 145, Kapitel 25 564, 1968) und wie in J. Clin. Pathol. (1979) 32, 971 beschrieben, für die indirekte Immunperoxidasetechnik weiterbehandelt werden.

Der durch seine Reaktivität mit verschiedenen Epitopen auf den 180 ± 10 KD und 95 ± 10 KD, grossen CEA-Molekülen bzw. auf CEA-verwandte Proteinen definierte monoklonale Antikörper nach der Erfindung kann zur diagnostischen Erkennung CEA-positiver Gewebe (z. B. Colonkarzinome, Pankreaskarzinome, Magenkarzinome, Mammakarzinome, Lungenkarzinome und Entzündungen) benutzt werden.

Auch können alle die oben beschriebenen Antigene erkennenden Antikörper, nach der Erfindung dazu benutzt werden, in radioaktiv markierter Form an die das jeweilige Antigen tragende Gewebe in vivo zu binden und die Antigene nachzuweisen (Immunszintigraphie).

Zusätzlich kann der erfindungsgemässe monoklonale Antikörper als Wirkstoffträger eingesetzt und zur Therapie maligner Erkrankungen benutzt werden. Eine weitere Anwendungsmöglichkeit stellt die Hemmung der Tumorzellmetastasierung nach in vivo-Applikation und Bindung der monoklonalen Antikörper an antigentragende metastasierende Tumorzellen dar. Ausserdem kann der monoklonale Antikörper ohne die Anwesenheit anderer Toxine für die antigentragenden Zellen toxisch sein oder nach Bindung an die Tumorzellenmembran zu Differenzierungsvorgängen führen, die bösartige Tumorzellen zu benignen Zellen werden lassen.

Für die diagnostischen Zwecke kann der monoklonale Antikörper je nach Test in einer Konzentration von 0,001 µg/ml bis 100 µg/ml eingesetzt werden.

Therapeutische Effekte werden in Mengen von 200 µg-100 mg/kg Körpergewicht erreicht.

## Patentansprüche

1. Monoklonaler Antikörper
**dadurch gekennzeichnet,**
**dass** er an CEA (Carcino embryonic antigen) und NCA₂ (Non specific reacting antigen) nicht aber an CEA_{low} und NCA₁ bindet und dessen Bindung an mit Perjodat und mit Neuraminidase behandelte sowie mit Formaldehyd fixierte und in Paraffin eingebettete Gewebe nicht beeinträchtigt ist und dass er durch Immunisierung von Säugetieren mit CEA und/oder Zellen eines CEA-positiven Adenokarzinoms herstellbar ist.

2. Monoklonaler Antikörper nach Anspruch 1,
**gekennzeichnet durch** seine Herstellung **durch** Immunisieren von Säugetieren mit CEA und/oder Zellen eines CEA-positiven Adenokarzinoms, und **durch** Entnehmen von Milzzellen dieser immunisierten Tiere und Fusionieren mit der Zelllinie X63AG8653 (American Type Culture Collection, ATCC Nr. CRL-1580) sowie **durch** Selektionieren der Hybridome zu den monoklonalen Antikörpern.

3. Monoklonaler Antikörper nach einem der Ansprüche 1 bis 2 **dadurch gekennzeichnet, dass** die Säugetiere Mäuse sind.

4. Monoklonaler Antikörper nach einem der Ansprüche 1 bis 3, **gekennzeichnet durch** seine Verwendung als Träger eines pharmazeutischen Wirkstoffes.

5. Monoklonaler Antikörper nach einem der Ansprüche 1 bis 3 in radiomarkierter Form zur Verwendung zum szintigraphischen Nachweis von Zellen und Geweben, die das Antigen exprimieren.

6. Monoklonaler Antikörper nach Anspruch 3 in radiomarkierter Form zur Verwendung zum Nachweis von entzündlichen Prozessen basierend auf der Bindung des Antikörpers an auf Granulozyten exprimiertem NCA-95.

## Claims

1. Monoclonal antibody
**characterized in that**
it binds to CEA (carcinoembryonic antigen) and NCA₂ (non-specific reacting antigen) but not to CEA_{low} and NCA₁ and its binding to tissue treated with periodate and with neuraminidase and to tissue fixed with formaldehyde and embedded in paraffin is not impaired and that it can be produced by immunizing mammals with CEA and/or cells of a CEA-positive adenocarcinoma.

2. Monoclonal antibody as claimed in claim 1,
**characterized by** its production by immunizing mammals with CEA and/or cells of a CEA-positive adenocarcinoma and by removing spleen cells from these immunized animals and fusing them with the cell line X63Ag8653 (American Type Culture Collection, ATCC No. CRL-1580) and by selecting the hybridomas for the monoclonal antibodies.

3. Monoclonal antibody according to one of the claims 1 to 2, **characterized in that** the mammals are mice.

4. Monoclonal antibody according to one of the claims 1 to 3, **characterized by** its use as a carrier of a pharmaceutically active substance.

5. Monoclonal antibody according to one of the claims 1 to 3 in a radiolabelled form for use in a scintigraphic detection of cells and tissues which express the antigen.

6. Monoclonal antibody according to claim 3 in a radiolabelled form for use in the detection of inflammatory processes based on the binding of the antibody to NCA-95 expressed on granulocytes.

## Revendications

1. Anticorps monoclonal, **caractérisé en ce qu'**il se lie au CEA (antigène carcinoembryonnaire) et au NCA₂ (antigène à réaction non spécifique), mais non au CEA_{low} ni au NCA₁, et sa liaison à un tissu traité avec du periodate et de la neuraminidase, ainsi que fixé avec du formaldéhyde et enrobé dans de la paraffine n'est pas inhibée, et **en ce qu'**il peut être préparé par immunisation de mammifères avec du CEA et/ou des cellules d'un adénocarcinome positif pour le CEA.

2. Anticorps monoclonal selon la revendication 1, **caractérisé en ce qu'**il est préparé par immunisation de mammifères avec du CEA et/ou des cellules d'un adénocarcinome positif pour le CEA, prélèvement de cellules spléniques de ces animaux immunisés, fusion avec la lignée cellulaire X63AG8653 (American Type Culture Collection, ATCC CRL-1580) et sélection des hybridomes produisant les anticorps monoclonaux.

3. Anticorps monoclonal selon l'une des revendications 1 à 2, **caractérisé en ce que** les mammifères sont des souris.

4. Anticorps monoclonal selon l'une des revendications 1 à 3, **caractérisé en ce qu'**il est utilisé comme véhicule d'une substance active pharmaceutique.

5. Anticorps monoclonal selon l'une des revendications 1 à 3 sous forme marquée par une substance radioactive pour l'utilisation dans la détection par scintigraphie de cellules et de tissus exprimant l'antigène.

6. Anticorps monoclonal selon la revendication 3 sous forme marquée par une substance radioactive pour l'utilisation dans la mise en évidence de processus inflammatoires fondée sur la liaison de l'anticorps au NCA-95 exprimé sur les granulocytes.
